# FASCICULE DE BREVET EUROPEEN

(11) **EP 0 498 724 B1**
(45) Date de publication et mention de la délivrance du brevet: **15.05.1996**
(21) Numéro de dépôt: 92400297.5
(22) Date de dépôt: 05.02.1992
(51) Int. Cl.: C07C 69/743, A01N 53/00, C07C 33/48

(54) **Nouveaux esters de l'acide 3-(3,3,3-trifluoro 2-chloropropényl) 2,2-diméthyl cyclopropanecarboxylique, leur procédé de préparation et leur application comme pesticides**
3-(3,3,3-Trifluor-2-Chlorpropenyl)-2,2-Dimethylcyclopropancarbonsäureester, Verfahren zu ihrer Herstellung und ihre Verwendung als Pestitzide
Esters of 3-(3,3,3-trifluoro-2-chloropropenyl)-2,2-dimethyl-cyclopropanecarboxylic acid, process for their preparation and their use as pesticides

(30) Priorité: 07.02.1991 FR 9101375
(43) Date de publication de la demande: 12.08.1992
(73) Titulaire: ROUSSEL UCLAF, F-93230 Romainville (FR)
(72) Inventeur: Benoit, Marc, F-13360 Roquevaire (FR); Demassey, Jacques, F-77144 Montevrain (FR); Demoute, Jean-Pierre, F-93360 Neuilly Plaisance (FR)
(74) Mandataire: Vieillefosse, Jean-Claude

(56) Documents cités:
- EP-A- 0 196 156
- EP-A- 0 271 240
- GB-A- 2 034 700
- GB-A- 2 120 655

## Description

### Nouveaux esters de l'acide 3-(3,3,3-trifluoro 2-chloro propényl) 2,2-diméthyl cyclopropanecarboxylique, leur procédé de préparation et leur application comme pesticides.

La présente invention concerne de nouveaux esters de l'acide 3-(3,3,3-trifluoro 2-chloro propényl) 2,2-diméthyl cyclopropanecarboxylique, leur procédé de préparation et leur application comme pesticides.

On connaissait déjà des dérivés polyfluorés du 2,2-diméthyl 3- (3,3,3-trifluoro2-chloro propenyl)cyclopropane corboxylate de benzyle doués de propriétés pesticides (cf GB-A 2120655 et GB-A 2034700).

L'invention a pour objet sous toutes leurs formes stéréoisomères possibles ainsi que leurs mélanges, les composés répondant à la formule générale (I) : 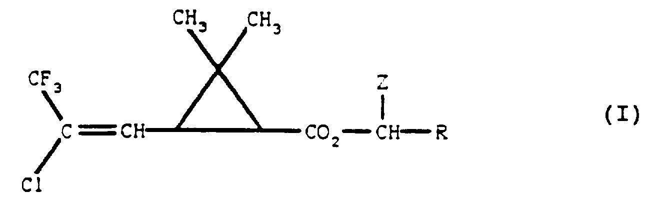 dans laquelle :
Z représente un atome d'hydrogène, un radical CH₃, un radical C≡N, ou un radical C≡CH,
et R représente un radical phényle substitué choisi dans le groupe constitué par les radicaux : 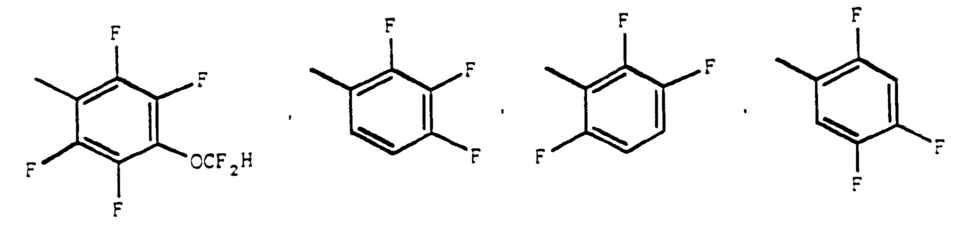 ET

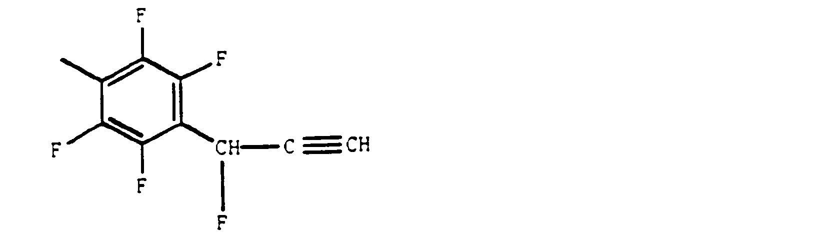

Les composés de formule (I) peuvent exister sous différentes formes stéréoisomères au niveau de la copule cyclopropanique. La géométrie de la double liaison peut être E ou Z ou un mélange de géométrie E et Z.

L'invention a pour objet les différents stéréoisomères possibles, ainsi que leurs mélanges.

L'invention a plus particulièrement pour objet les composés de formule (I) dans lesquels la structure cyclopropanique est 1R [cis], ainsi que ceux dans lesquels la géométrie de la double liaison est Z.

Parmi les composés préférés de l'invention, on peut citer les composés de formule (I) dans laquelle Z représente un atome d'hydrogène.

L'invention a plus particulièrement pour objet les composés de formule (I) dont la préparation est donnée ci-après, dans la partie expérimentale, et dont les noms suivent :
- 1R [1alpha, 3alpha (Z)] 2,2-diméthyl 3-(3,3,3-trifluoro 2-chloro propényl) cyclopropanecarboxylate de [4-(difluorométhoxy) 2,3,5,6-tétrafluorophényl] méthyle,
- 1R [1alpha, 3alpha (Z)] 2,2-diméthyl 3-(3,3,3-trifluoro 2-chloro propényl) cyclopropanecarboxylate de (2,3,4-trifluorophényl) méthyle,
- 1R [1alpha, 3alpha (Z)] 2,2-diméthyl 3-(3,3,3-trifluoro 2-chloro propényl) cyclopropanecarboxylate de (2,3,6-trifluorophényl) méthyle,
- 1R [1alpha, 3alpha (Z)] 2,2-diméthyl 3-(3,3,3-trifluoro 2-chloro propényl) cyclopropanecarboxylate de (2,4,5-trifluorophényl) méthyle,
- 1R [1alpha, 3alpha (Z)] 2,2-diméthyl 3-(3,3,3-trifluoro 2-chloro propényl) cyclopropanecarboxylate de [[(4-(1-fluoro 2-propynyl)] 2,3,5,6-tétrafluoro phényl] méthyle.

L'invention a aussi plus particulièrement pour objet les composés de formule (I) dont les noms suivent :
- 1R [1alpha, 3alpha (E + Z)] 2,2-diméthyl 3-(3,3,3-tridlurod 2-chloropropényl) cyclopropane carboxylate de (2,3,6-trifluorophényl) méthyle.

L'invention a également pour objet un procédé de préparation des composés de formule (I), caractérisé en ce que l'on soumet un acide de formule (II) : 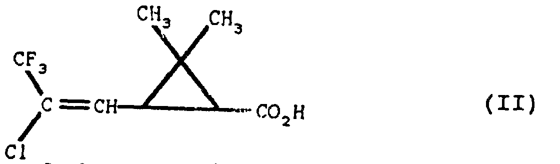 ou un dérivé fonctionnel de cet acide à l'action d'un alcool de formule (III) : 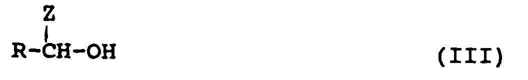 dans laquelle R et Z conservent leur signification précédente ou d'un dérivé fonctionnel de cet alcool pour obtenir le composé de formule (I) correspondant.

L'estérification de l'acide de formule (II) avec l'alcool de formule (III) peut être effectuée en présence d'un agent déshydratant, d'une base tertiaire, telle que la pyridine. Cette estérification peut être effectuée avantageusement en présence d'un mélange de dicyclohexylcarbodiimide et de pyridine ou de 4-diméthyl aminopyridine.

L'estérification peut également être réalisée en faisant réagir un chlorure d'acide (II) avec l'alcool (III) ou avec un dérivé organo-métallique de cet alcool.

Dans un autre mode de préparation, l'estérification de l'acide de formule (II) peut être réalisée en faisant réagir l'acide ou l'un de ses sels avec un dérivé halogéné (IV) : 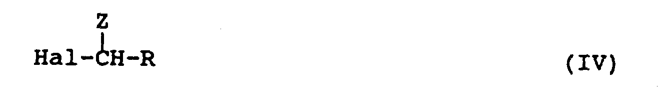 dans laquelle Z et R ont la signification indiquée ci-dessus ou avec un ester activé de l'alcool (III).

L'alcool 4-difluorométhoxy 2,3,5,6-tétrafluorobenzylique est un produit connu qui peut être préparé selon le procédé décrit dans la demande de brevet européen 0281439 (cf préparation 6).

Les alcools trifluorobenzyliques utilisés peuvent être préparés à partir des acides correspondants.

L'alcool [4-(1-fluoro 2-propynyl) 2,3,5,6-tétrafluoro] benzylique est un produit nouveau dont la préparation est donnée ci-après dans la partie expérimentale.

L'invention a également pour objet cet alcool, à titre de produit chimique nouveau.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la préparation des compositions pesticides destinées à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères comme Diabrotica, les taupins et les vers blancs, les myriapodes comme les scutigérelles et les blaniules, les diptères comme les cécydomies et les lépidoptères comme les noctuelles terricoles.

Ils sont utilisés à des doses comprises entre 10 g et 300 g de matière active à l'hectare.
Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait que les produits de formule (I) correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce de Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment les produits de formule (I) des exemples 1, 2, 3, 4, 5 ou 6.

L'invention a notamment pour objet les compositions insecticides renfermant comme principe actif au moins l'un des produits définis ci-dessus.

L'invention a particulièrement pour objet les compositions insecticides définies ci-dessus destinées à la lutte contre Diabrotica et autres parasites du sol.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides et nématicides renfermant comme principe actif au moins un des produits de formule (I) définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo [2,2-1] 5-heptène-2,3-dicarboximide, ou le pipéronyl-bis-2-(2′-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcools 3-phénoxy benzylique et d'alcools alpha-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcools alpha-cyano 3-phénoxy benzyliques des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention sans toutefois la limiter.

### EXEMPLE 1 : 1R[1alpha, 3alpha (Z)] 2,2-diméthyl 3-(3,3,3-trifluoro 2-chloro propényl) cyclopropanecarboxylate de [4-(difluorométhoxy) 2,3,5,6-tétrafluorophényl] méthyle,

On dissout sous atmosphère d'azote, 1,45 g d'acide 1R [1alpha, 3alpha (Z)] 2,2-diméthyl 3-(3,3,3-trifluoro 2-chloro propényl) cyclopropanecarboxylique et 1,48 g de 2,3,5,6-tétrafluoro 4-(difluorométhoxy) phénylhydroxyméthyle obtenu selon EP 0281439, dans 10 cm**³** de chlorure de méthylène. On refroidit à 0°/-10°C et introduit en 15 minutes une solution renfermant 1,24 g de dicyclohexylcarbodiimide, 14 mg de 4-diméthylaminopyridine et 8 cm**³** de chlorure de méthylène. On maintient la suspension obtenue sous agitation à la température ambiante pendant 1 heure. On filtre, rince à l'éther isopropylique et amène à sec. On chromatographie le résidu obtenu sur silice en éluant avec le mélange hexane-éther isopropylique (9-1). On obtient ainsi 2,62 g de produit recherché.
[alpha]_{**D**} = +27,5° ± 2° (c = 0,5 % CHCl₃)
Analyse pour C₁₇H₁₂ClF₉O₃ = 470,723

| | | | | |
|---|---|---|---|---|
| Calculé : | C% 43,38 | H% 2,57 | Cl% 7,53 | F% 36,32 |
| Trouvé : | 43,5 | 2,4 | 7,7 | 36,3 |

En opérant comme précédemment, à partir du même acide et des alcools appropriés, on a obtenu les produits des exemples suivants.

### EXEMPLE 2 : 1R [1alpha, 3alpha (Z)] 2,2-diméthyl 3-(3,3,3-trifluoro 2-chloro propényl) cyclopropanecarboxylate de (2,3,4-trifluorophényl) méthyle,

[alpha]_{**D**} = +15,5° (c = 0,9 % toluène)

### EXEMPLE 3 : 1R [1alpha, 3alpha (Z)] 2,2-diméthyl 3-(3,3,3-trifluoro 2-chloro propényl) cyclopropanecarboxylate de (2,3,6-trifluorophényl) méthyle,

[alpha]_{**D**} = +6° (c = 0,5 % toluène)

### EXEMPLE 4 : 1R [1alpha, 3alpha (Z)] 2,2-diméthyl 3-(3,3,3-trifluoro 2-chloro propényl) cyclopropanecarboxylate de (2,4,5-trifluorophényl) méthyle,

[alpha]_{**D**} = +26° (c = 0,75 % toluène)

### EXEMPLE 5 : 1R [1alpha, 3alpha (Z)] 2,2-diméthyl 3-(3,3,3-trifluoro 2-chloro propényl) cyclopropanecarboxylate de [[(4-(1-fluoro 2-propynyl)] 2,3,5,6-tétrafluoro phényl] méthyle. [alpha]_{D} = +16° (c = 1 % CHCl₃)

Spectre de RMN (CDCl**₃**) ppm
- CH**₃**: 1,3 (s)
- proton cyclopropane: 1,89 - 2,36 (m)
- C ≡ CH: 2,87
- CH**₂**-O: 5,23
- CH - F: 6,40
- =CH: 6,82

En opérant comme précédemment à partir de l'acide 1R cis (E + Z) et de l'alcool approprié, on a obtenu le produit suivant :

### EXEMPLE 6 : (1R)-[1alpha, 3alpha (E + Z)] 2,2-diméthyl-3-(3,3,3-trifluoro-2-chloropropényl) cyclopropanecarboxylate de (2,3,6-trifluorophényl) méthyle.

Spectre de RMN (CDCl₃) ppm
- CH₃ (gem) :: 1,24-1,29 (s)
- H₃ cyclopropane :: 2,18 (m)
- H₁ delta E :: 1,90 (d, J=9,5)
- H₁ delta Z :: 1,98 (d, J=9,5)
- CH₂-O :: 5,21 (m)
- >=CH delta E ¦: 6,61 (d, J=9,5)
- delta Z ¦: 6,90 (d, J=9,5)

### Préparation 1 : Alcool [4-(1-fluoro 2-propynyl) 2,3,5,6-tétrafluoro] benzylique

### Stade A : [1-(tétrahydro 2H-pyran-2-yl) oxy] [2,3,5,6-tétrafluoro 4-carboxaldéhyde phényl] méthyle.

On refroidit à -60°C 13 g de [[1-(tétrahydro 2H-pyran-2-yl) oxy] 2,3,5,6-tétrafluorophényl] méthyle en solution dans 200 cm³ de tétrahydrofuranne. On ajoute goutte à goutte en une demi heure 35,1 cm³ de butyl lithium en solution 1,6 M dans l'hexane. On maintient sous agitation pendant 1 heure à -55°C. On ajoute ensuite en 5 minutes à -55°C 5 cm³ de diméthylformamide en solution dans 10 cm³ de tétrahydrofuranne. On maintient sous agitation pendant 1 h 30 à -55°C et traite avec 50 cm³ de phosphate acide de sodium. On extrait à l'éther, sèche et concentre. On obtient 14,4 g de produit recherché.
Spectre IR: (CHCl₃)
- C=O: 1709 cm⁻¹
- Aromatique: 1655 - 1490 cm⁻¹

### Stade B : [1-(tétrahydro 2H-pyran-2-yl) oxy] [2,3,5,6-tétrafluoro 4-(1-hydroxy 2-triméthyl silyle propynyl) phényl] méthyle

On verse à -78°C 12 cm³ de triméthylsilyl acétylène dans 120 cm³ de tétrahydrofuranne. On ajoute 34 cm³ d'une solution 1,6 M de n-butyl lithium dans l'hexane. On laisse remonter la température à -20°C. On ajoute 15 g de [l-(tétrahydro 2H-pyran-2-yl) oxy] [2,3,5,6-tétrafluoro 4-carboxaldéhyde phényl] méthyle obtenu au stade A dans 30 cm³ de tétrahydrofuranne. On agite pendant 3 heures entre -20°C et -30°C et verse lentement sur une solution aqueuse saturée de phosphate acide de potassium. On décante, extrait à l'éther et évapore à sec. On cristallise dans le pentane sous ultra-sons. On filtre et lave avec du pentane. On obtient 8,58 g de produit fondant à 102°C. On chromatographie les liqueurs mères sur silice en éluant avec le mélange hexane-éther diisopropylique toluène (3-1-6). On obtient 9,55 g de produit attendu supplémentaire soit 18,13 g au total.

### Stade C : [1-(tétrahydro 2H-pyran-2-yl) oxy] [2,3,5,6-tétrafluoro 4-(1-hydroxy 2-propynyl) phényl] méthyle

On dissout 0,39 g de produit préparé au stade précédent dans 6 cm³ de méthanol. On ajoute 40 mg de carbonate de potassium. On maintient sous agitation le mélange réactionnel pendant 2 heures et ajoute 0,3 cm³ d'une solution d'acide chlorhydrique 1N. On évapore à sec, reprend à l'éther et lave à l'eau. On sèche et évapore. On obtient 0,34 g de produit utilisé tel quel au stade suivant.

### Stade D : [1-(tétrahydro 2H-pyran-2-yl) oxy] [2,3,5,6-tétrafluoro 4-(1-fluoro 2-propynyl) phényl] méthyle

On met le produit précédent en solution dans 5 cm³ de chlorure de méthylène, sous atmosphère d'azote à -78°C. On ajoute 0,15 cm³ de trifluorodiméthylaminosulfure. On verse sur une solution aqueuse de bicarbonate de soude. On décante, extrait au chlorure de méthylène, sèche et évapore à sec. On chromatographie sur silice avec le mélange hexane-acétate d'éthyle (95-5). On obtient 0,22 g de produit recherché.
Spectre de RMN (CDCl₃, 300 MHz) ppm
- C-CH₂-C du tétrahydropyranyle: 1,45 - 1,90 (m)
- C ≡ CH: 2,89 (dd,j = 2,5 et 5,5 Hz)
- CH₂-O du tétrahydropyranyle: 3,56 - 3,89 (m)
4,61 (d,j = 11)
- Ar-CH₂-O: 4,86 (d,j = 11)
- O-CH-O du tétrahydropyranyle: 4,78 (m)
- CH-F: 6,38 (dd,j = 2,5 et 45,5 Hz)

### Stade E : Alcool [4-(1-fluoro 2-propynyl)] 2,3,5,6-tétrafluoro benzylique

On dissout 2,24 g du produit préparé au stade précédent dans 30 cm³ de méthanol. On ajoute 0,1 g d'acide paratoluène sulfonique. On maintient sous agitation pendant 1 heure à la température ambiante. On neutralise à l'aide d'une solution de soude 2N. On évapore à sec. On reprend au chlorure de méthyet lave à l'eau. On chromatographie sur silice en éluant avec du chlorure de méthylène. On obtient 1,31 g de produit recherché.
Spectre de RMN (CDCl**₃**, 300 MHz)
- C ≡ CH: 2,89 (dd,j = 2 et 5 Hz)
- CH**₂**-O: 4,85 (s)
- CH-F: 6,37 (dm, j_{**H-F**} = 45 Hz)
- OH: 2,28 (s)

### Préparation 2 : Alcool 2,3,6-trifluoro benzylique

On dissout à la température ambiante, sous atmosphère d'azote 10,56 g d'acide 2,3,6-trifluorobenzoïque dans 100 cm**³** de tétrahydrofuranne. On refroidit au bain de glace-méthanol et ajoute en 30 minutes une solution renfermant 10 cm**³** de complexe borane méthyl sulfure à 10 millimoles/cm**³** et 30 cm**³** de tétrahydrofuranne. On agite encore pendant 5 minutes et laisse revenir à la température ambiante. On chauffe pendant 3 h 30 à 45^{∿} 50°C. On verse la solution obtenue sur une solution aqueuse de phosphate acide de sodium. On extrait à l'éther isopropylique, lave, sèche et amène à sec. On obtient 10,6 g de liquide incolore que l'on chromatographie sur silice en éluant avec le mélange hexane-acétate d'éthyle (7-3). On obtient 8,8 g de produit recherché. F < 50°C.
Spectre IR (CHCl**₃**)
- OH: 3620 cm**⁻¹**
- Aromatique: 1642, 1604, 1499 cm**⁻¹**
Spectre de RMN (CDCl**₃**, 60 Hz) ppm
- Protons aromatiques: 6,70 - 7,47
- CH**₂**-OH: 4,83
- OH: 2,20

### Préparation 3 : Alcool 2,4,5-trifluoro benzylique

En opérant comme précédemment à partir de 8 g de l'acide correspondant, on a obtenu 6,85 g du produit recherché.
Spectre IR (CHCl**₃**)
- OH: 3612 cm**⁻¹**
1634 cm**⁻¹** (m)
- Aromatique: 1520 cm**⁻¹** (F)

### Préparation 4 : Alcool 2,3,4-trifluoro benzylique

En opérant comme à la préparation 2, à partir de 5 g d'acide correspondant, on a obtenu 4,39 g de produit recherché.
Spectre IR

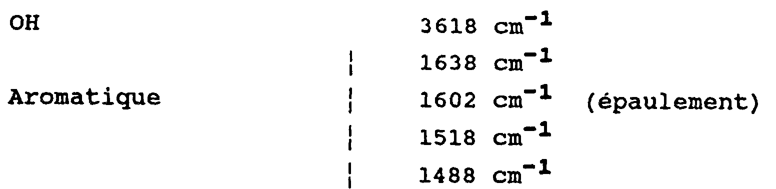

### EXEMPLE 7 : Préparation d'un concentré soluble

On effectue un mélange homogène de :
- Produit de l'exemple 1 :: 0,25 g
- Butoxyde pipéronyle :: 1,00 g
- Tween 80 :: 0,25 g
- Topanol A :: 0,1 g
- Eau :: 98,4 g

### EXEMPLE 8 : Préparation d'un concentré émulsifiable

On mélange intimement :
- Produit de l'exemple 2 :: 0,015 g
- Butoxyde de pipéronyle :: 0,5 g
- Topanol A :: 0,1 g
- Tween 80 :: 3,5 g
- Xylène :: 95,885 g

### EXEMPLE 9 : Préparation d'un concentré émulsifiable

On effectue un mélange homogène de :
- Produit de l'exemple 3 :: 1,5 g
- Tween 80 :: 20,00 g
- Topanol A :: 0,1 g
- Xylène :: 78,4 g

### EXEMPLE 10 : Préparation de granulés

On a préparé des granulés contenant de 0,1 % à 5 % de substances actives.

### ETUDE BIOLOGIQUE

### A - Activité sur Diabrotica

Les insectes tests sont des larves de dernier stade de Diabrotica.

On traite une rondelle de papier filtre de 9 cm de diamètre, déposée au fond d'une boîte de Pétri, à l'aide de 2 cm³ de solution acétonique du produit à tester. Après séchage on dépose 10 larves par dose et on effectue le contrôle de mortalité 24 heures après le traitement. On effectue plusieurs tests, selon différentes concentrations du produit à tester.

Dès la dose de 0,5 ppm les produits de l'invention présentent une bonne activité.

### B - Etude de l'effet d'abattage sur mouche domestique

Les insectes tests sont des mouches domestiques femelles âgées de 4 jours. On opère par pulvérisation en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

A la dose de 1 g/l les produits de l'invention présentent une bonne activité.

### C - Etude de l'effet létal sur mouche domestique

Les insectes tests sont des mouches domestiques femelles âgées de 4 à 5 jours. On opère par application topique de 1 microlitre de solution acétonique du produit à tester sur le thorax dorsal des insectes à l'aide du micro manipulateur d'Arnold. On utilise 50 individus par traitement. On effectue le contrôle de mortalité vingt-quatre heures après traitement.

Dès la dose de 10 mg/l les produits de l'exemple 1 et 5 présentent une bonne activité.

### D - Etude de l'effet létal sur larves de Spodoptera Littoralis

Les essais sont effectués par application topique d'une solution acétonique du produit à tester, à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24°C et 65 % d'humidité relative. Après traitement les individus sont placés sur milieu nutritif artificiel (milieu de Poitout).

On effectue le contrôle des mortalités 48 heures après traitement.

Dès la dose de 5 mg/l les produits de l'exemple 1 et 5 présentent une bonne activité.

## Revendications (Revendications pour l'(les) Etat(s) contractant(s) suivant(s): AT, BE, CH, DE, DK, FR, GB, GR, IT, LI, LU, MC, NL, SE)

1. Sous toutes les formes stéréoisomères possibles ainsi que leurs mélanges, les composés répondant à la formule générale (I) : 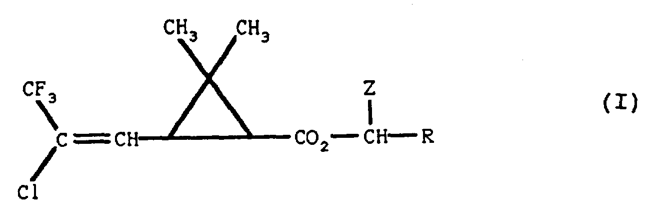 dans laquelle :
Z représente un atome d'hydrogène, un radical CH₃, un radical C≡N, ou un radical C≡CH,
et R représente un radical phényle substitué choisi dans le groupe constitué par les radicaux : 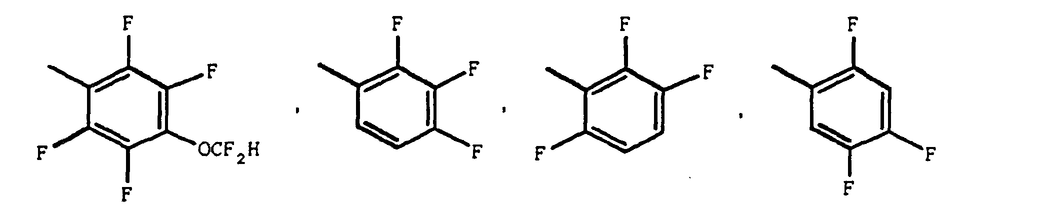 ET

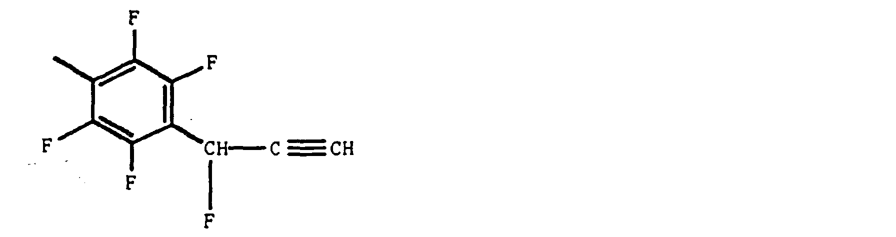

2. Les composés de formule (I) tels que définis à la revendication 1 dans lesquels la structure cyclopropanique est de structure 1R [cis].

3. Les composés de formule (I) selon la revendication 1 dans lesquels la géométrie de la double liaison est Z.

4. Les composés de formule (I) selon la revendication 1 dans lesquels Z représente un atome d'hydrogène.

5. Les composés de formule (I) tels que définis à la revendication 1 dont les noms suivent :
- 1R [1alpha, 3alpha (Z)] 2,2-diméthyl 3-(3,3,3-trifluoro 2-chloro propényl) cyclopropanecarboxylate de [4-(difluorométhoxy) 2,3,5,6-tétrafluorophényl] méthyle,
- 1R [1alpha, 3alpha (Z)] 2,2-diméthyl 3-(3,3,3-trifluoro 2-chloro propényl) cyclopropanecarboxylate de (2,3,4-trifluorophényl) méthyle,
- 1R [1alpha, 3alpha (Z)] 2,2-diméthyl 3-(3,3,3-trifluoro 2-chloro propényl) cyclopropanecarboxylate de (2,3,6-trifluorophényl) méthyle,
- 1R [1alpha, 3alpha (Z)] 2,2-diméthyl 3-(3,3,3-trifluoro 2-chloro propényl) cyclopropanecarboxylate de (2,4,5-trifluorophényl) méthyle,
- 1R [1alpha, 3alpha (Z)] 2,2-diméthyl 3-(3,3,3-trifluoro 2-chloro propényl) cyclopropanecarboxylate de [[(4-(1-fluoro 2-propynyl)] 2,3,5,6-tétrafluoro phényl] méthyle,
- 1R [1alpha, 3alpha (E + Z)] 2,2-diméthyl-3-(3,3,3-trifluoro-2-chloropropényl) cyclopropanecarboxylate de (2,3,6-trifluorophényl) méthyle.

6. Procédé de préparation des composés de formule (I) tels que définis à l'une quelconque des revendications 1 à 5, caractérisé en ce que l'on soumet un acide de formule (II) : 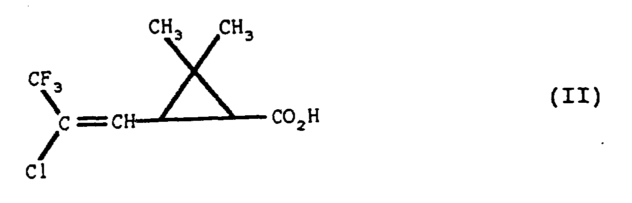 ou un dérivé fonctionnel de cet acide à l'action d'un alcool de formule (III) : 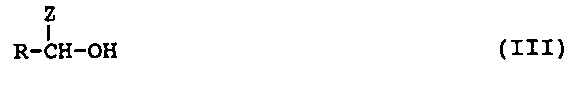 dans laquelle R et Z conservent leur signification précédente ou d'un dérivé fonctionnel de cet alcool pour obtenir le composé de formule (I) correspondant.

7. Le composé de formule (III) tel que défini à la revendication 6 dont le nom suit :
- alcool [4-(1-fluoro 2-propynyl) 2,3,5,6-tétrafluoro] benzylique.

8. Application des composés de formule (I) tels que définis à l'une quelconque des revendications 1 à 5, à la préparation des compositions pesticides destinées à la lutte contre les parasites des végétaux, les parasites des locaux et les parasites des animaux à sang chaud.

9. Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 5.

10. Les compositions insecticides, caractérisées en ce qu'elles renferment comme principe actif au moins un composé défini à l'une quelconque des revendications 1 à 5.

11. Les compositions insecticides définies à la revendication 10, caractérisées en ce qu'elles sont destinées à la lutte contre DIABROTICA et les autres parasites du sol.

12. Les compositions acaricides, caractérisées en ce qu'elles renferment comme principe actif au moins un composé défini à l'une quelconque des revendications 1 à 5.

13. Les compositions définies à l'une quelconque des revendications 9 à 12 caractérisées en ce qu'elles renferment comme principe actif au moins un composé défini à la revendication 5.

14. Associations douées d'activité insecticide, acaricide ou nématicide, caractérisées en ce qu'elles contiennent comme matière active, d'une part, un au moins des composés de formule générale (I) tels que définis à la revendication 1 et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachloropnényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

## Revendications (Revendications pour l'(les) Etat(s) contractant(s) suivant(s): ES)

1. Procédé pour préparer sous toutes les formes stéréoisomères possibles ainsi que leurs mélanges, les composés répondant à la formule générale (I) : 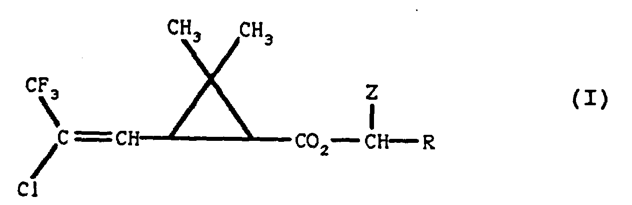 dans laquelle :
Z représente un atome d'hydrogène, un radical CH₃, un radical C≡N, ou un radical C≡CH,
et R représente un radical phényle substitué choisi dans le groupe constitué par les radicaux : 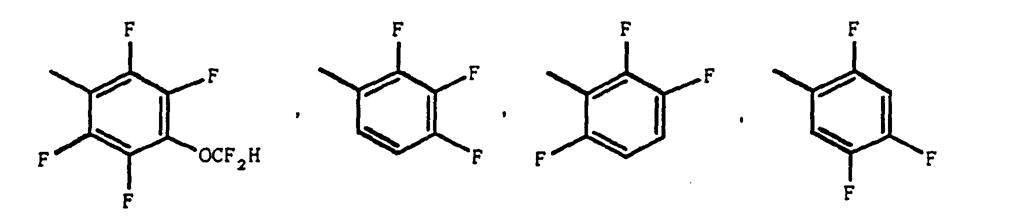 ET 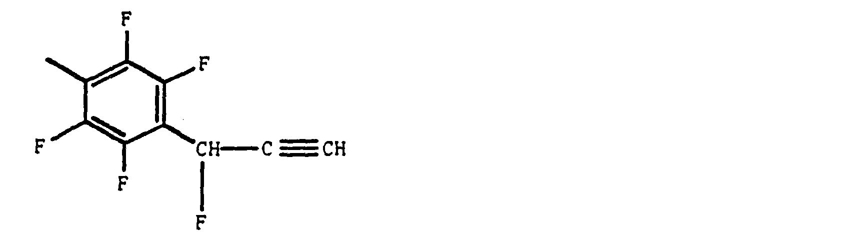 caractérisé en ce que l'on soumet un acide de formule (II) : 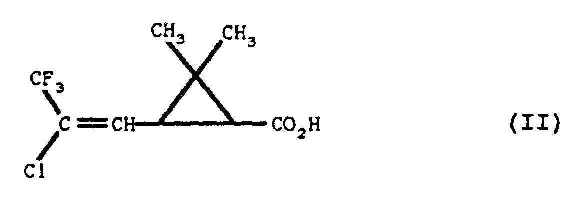 ou un dérivé fonctionnel de cet acide à l'action d'un alcool de formule (III) : 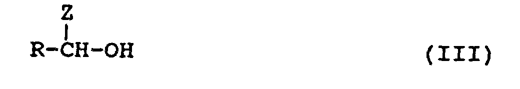 dans laquelle R et Z conservent leur signification précédente ou d'un dérivé fonctionnel de cet alcool pour obtenir le composé de formule (I) correspondant.

2. Procédé selon la revendication 1, caractérisé en ce que l'on utilise au départ un acide de formule (II) dans laquelle la structure cyclopropanique est de structure 1R [cis].

3. Procédé selon la revendication 1 ou 2, caractérisé en ce que l'on utilise au départ un acide de formule (II) dans laquelle la géométrie de la double liaison est Z.

4. Procédé selon l'une quelconque des revendications 1 à 3, caractérisé en ce que l'on utilise au départ un alcool de formule (III) dans laquelle Z représente un atome d'hydrogène.

5. Procédé selon l'une quelconque des revendications 1 à 4, caractérisé en ce que l'on soumet l'acide 1R [1alpha, 3alpha (Z)] 2,2-diméthyl 3-cyclopropane carboxylique à l'action d'un alcool de formule (III) approprié ou un dérivé fonctionnel de cet alcool pour obtenir l'un des composés de formule (I) dont les noms suivent :
- 1R [1alpha, 3alpha (Z)] 2,2-diméthyl 3-(3,3,3-trifluoro 2-chloro propényl) cyclopropanecarboxylate de [4-(difluorométhoxy) 2,3,5,6-tétrafluorophényl] méthyle,
- 1R [1alpha, 3alpha (Z)] 2,2-diméthyl 3-(3,3,3-trifluoro 2-chloro propényl) cyclopropanecarboxylate de (2,3,4-trifluorophényl) méthyle,
- 1R [1alpha, 3alpha (Z)] 2,2-diméthyl 3-(3,3,3-trifluoro 2-chloro propényl) cyclopropanecarboxylate de (2,3,6-trifluorophényl) méthyle,
- 1R [1alpha, 3alpha (Z)] 2,2-diméthyl 3-(3,3,3-trifluoro 2-chloro propényl) cyclopropanecarboxylate de (2,4,5-trifluorophényl) méthyle,
- 1R [1alpha, 3alpha (Z)] 2,2-diméthyl 3-(3,3,3-trifluoro 2-chloro propényl) cyclopropanecarboxylate de [[(4-(1-fluoro 2-propynyl)] 2,3,5,6-tétrafluoro phényl] méthyle.

## Revendications (Revendications pour l'(les) Etat(s) contractant(s) suivant(s): PT)

1. Procédé pour préparer sous toutes les formes stéréoisomères possibles ainsi que leurs mélanges, les composés répondant à la formule générale (I) : 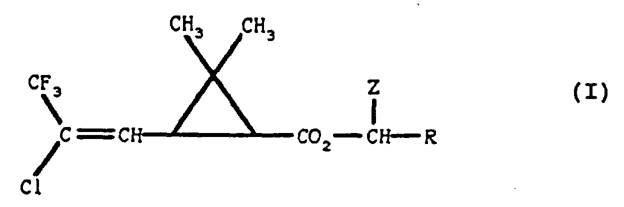 dans laquelle :
Z représente un atome d'hydrogène, un radical CH₃, un radical C≡N, ou un radical C≡CH,
et R représente un radical phényle substitué choisi dans le groupe constitué par les radicaux : 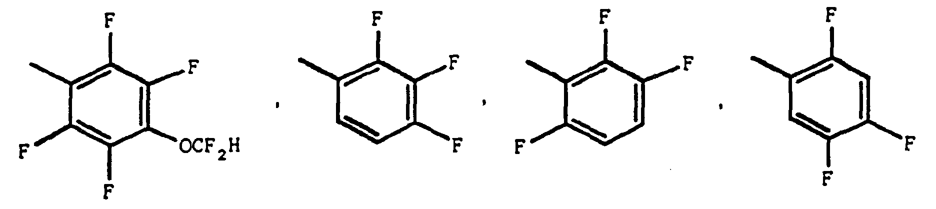 ET 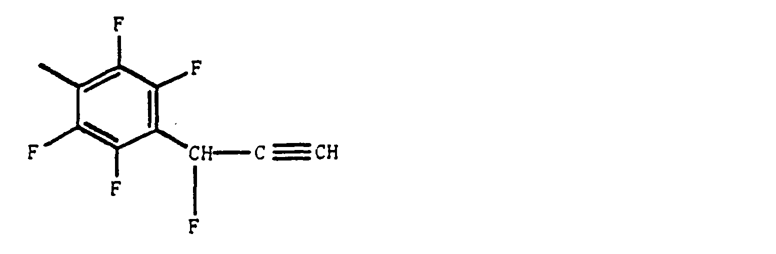 caractérisé en ce que l'on soumet un acide de formule (II) : 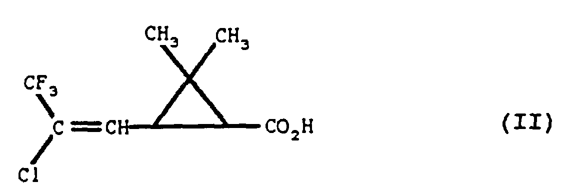 ou un dérivé fonctionnel de cet acide à l'action d'un alcool de formule (III) : 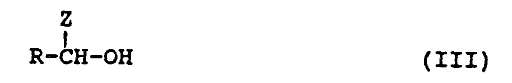 dans laquelle R et Z conservent leur signification précédente ou d'un dérivé fonctionnel de cet alcool pour obtenir le composé de formule (I) correspondant.

2. Procédé selon la revendication 1, caractérisé en ce que l'on utilise au départ un acide de formule (II) dans laquelle la structure cyclopropanique est de structure 1R [cis].

3. Procédé selon la revendication 1 ou 2, caractérisé en ce que l'on utilise au départ un acide de formule (II) dans laquelle la géométrie de la double liaison est Z.

4. Procédé selon l'une quelconque des revendications 1 à 3, caractérisé en ce que l'on utilise au départ un alcool de formule (III) dans laquelle Z représente un atome d'hydrogène.

5. Procédé selon l'une quelconque des revendications 1 à 4, caractérisé en ce que l'on soumet l'acide 1R [1alpha, 3alpha (Z)] 2,2-diméthyl 3-cyclopropane carboxylique à l'action d'un alcool de formule (III) approprié ou un dérivé fonctionnel de cet alcool pour obtenir l'un des composés de formule (I) dont les noms suivent :
- 1R [1alpha, 3alpha (Z)] 2,2-diméthyl 3-(3,3,3-trifluoro 2-chloro propényl) cyclopropanecarboxylate de [4-(difluorométhoxy) 2,3,5,6-tétrafluorophényl] méthyle,
- 1R [1alpha, 3alpha (Z)] 2,2-diméthyl 3-(3,3,3-trifluoro 2chloro propényl) cyclopropanecarboxylate de (2,3,4-trifluorophényl) méthyle,
- 1R [1alpha, 3alpha (Z)] 2,2-diméthyl 3-(3,3,3-trifluoro 2-chloro propényl) cyclopropanecarboxylate de (2,3,6-trifluorophényl) méthyle,
- 1R [1alpha, 3alpha (Z)] 2,2-diméthyl 3-(3,3,3-trifluoro 2-chloro propényl) cyclopropanecarboxylate de (2,4,5-trifluorophényl) méthyle,
- 1R [1alpha, 3alpha (Z)] 2,2-diméthyl 3-(3,3,3-trifluoro 2-chloro propényl) cyclopropanecarboxylate de [[(4-(1-fluoro 2-propynyl)] 2,3,5,6-tétrafluoro phényl] méthyle.

6. Application des composés de formule (I) tels que définis à l'une quelconque des revendications 1 à 5, à la lutte contre les parasites des végétaux, les parasites des locaux et les parasites des animaux à sang chaud.

7. Procédé pour préparer des compositions destinées à la lutte contre les parasites des végétaux, des locaux et des animaux à sang chaud, caractérisé en ce que l'on met un au moins des composés de formule (I) tels que définis à l'une quelconque des revendications 1 à 5 sous une forme destinée à cet usage.

8. Procédé pour préparer des compositions insecticides, caractérisé en ce que l'on met un au moins des composés de formule (I) tels que définis à l'une quelconque des revendications 1 à 5 sous une forme destinée à cet usage.

9. Procédé pour préparer des compositions insecticides, destinées à la lutte contre Diabrotica et les autres parasites du sol, caractérisé en ce que l'on met un au moins des composés de formule (I) tels que définis à l'une quelconque des revendications 1 à 5 sous une forme destinée à cet usage.

10. Procédé pour préparer des compositions acaricides, caractérisé en ce que l'on met un au moins des composés de formule (I) tels que définis à l'une quelconque des revendications 1 à 5 sous une forme destinée à cet usage.

11. Procédé pour préparer des compositions selon l'une quelconque des revendications 7 à 10, caractérisé en ce que l'on met un au moins des composés de formule (I) tels que définis à la revendication 5 sous une forme destinée à cet usage.

12. Procédé de préparation de compositions douées d'activité insecticide, acaricide ou nématicide, caractérisé en ce que l'on mélange un au moins des composés de formule (I) telle que définie à l'une quelconque des revendications 1 à 5 avec un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

## Patentansprüche (Patentansprüche für folgende(n) Vertragsstaat(en): AT, BE, CH, DE, DK, FR, GB, IT, LI, LU, MC, NL SE)

1. In allen möglichen stereoisomeren Formen sowie ihren Mischungen die Verbindungen der allgemeinen Formel (I) 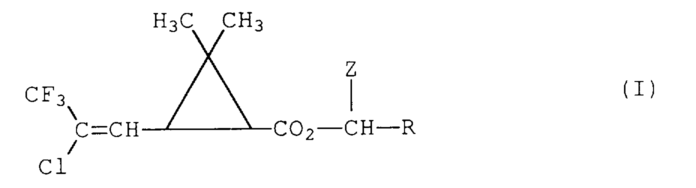 in der
Z ein Wasserstoffatom, einen Rest CH₃, einen Rest C≡N oder einen Rest C≡CH darstellt,
und R einen substituierten Phenylrest bedeutet, gewählt aus der durch die folgenden Reste gebildeten Gruppe: 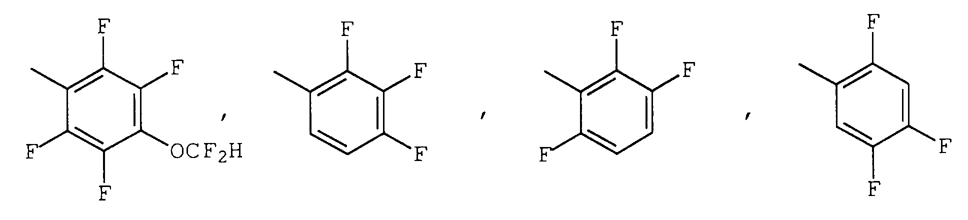 und

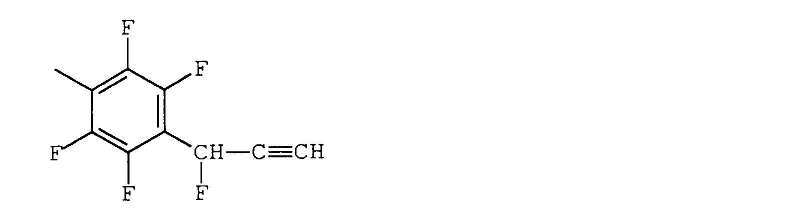

2. Verbindungen der Formel (I) wie in Anspruch 1 definiert, worin die Cyclopropan-Struktur die Struktur 1R [cis] besitzt.

3. Verbindungen der Formel (I) nach Anspruch 1, worin die Geometrie der Doppelbindung Z ist.

4. Verbindungen der Formel (I) nach Anspruch 1, worin Z ein Wasserstoffatom darstellt.

5. Verbindungen der Formel (I) wie in Anspruch 1 definiert, mit den folgenden Namen:
- 1R[1α,3α(Z)]-2,2-Dimethyl-3-(3,3,3-trifluor-2-chlor-propenyl)-cyclopropancarbonsäure-[4-(difluormethoxy)-2,3,5,6-tetrafluorphenyl]-methylester,
- 1R[1α,3α(Z)]-2,2-Dimethyl-3-(3,3,3-trifluor-2-chlor-propenyl)-cyclopropancarbonsäure-(2,3,4-trifluorphenyl)-methylester,
- 1R[1α,3α(Z)]-2,2-Dimethyl-3-(3,3,3-trifluor-2-chlor-propenyl)-cyclopropancarbonsäure-(2,3,6-trifluorphenyl)-methylester,
- 1R[1α,3α(Z)]-2,2-Dimethyl-3-(3,3,3-trifluor-2-chlor-propenyl)-cyclopropancarbonsäure-(2,4,5-trifluorphenyl)-methylester,
- 1R[1α,3α(Z)]-2,2-Dimethyl-3-(3,3,3-trifluor-2-chlor-propenyl)-cyclopropancarbonsäure-{[4-(1-fluor-2-propinyl)]-2,3,5,6-tetrafluorphenyl}-methylester,
- 1R[1α,3α(E+Z)]-2,2-Dimethyl-3-(3,3,3-trifluor-2-chlor-propenyl)-cyclopropancarbonsäure-(2,3,6-trifluorphenyl)-methylester.

6. Verfahren zur Herstellung der Verbindungen der Formel (I) wie in irgendeinem der Ansprüche 1 bis 5 definiert, dadurch gekennzeichnet, daß man eine Säure der Formel (II) 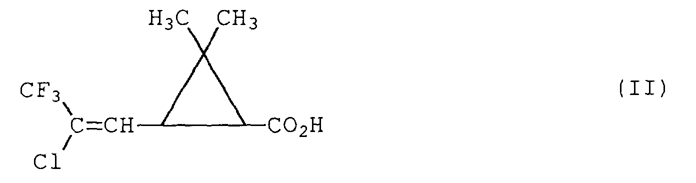 oder ein funktionelles Derivat dieser Säure der Einwirkung eines Alkohols der Formel (III) 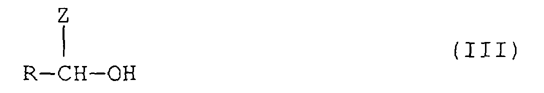 in der R und Z ihre oben angegebene Bedeutung behalten, oder eines funktionellen Derivates dieses Alkohols unterzieht, um die entsprechende Verbindung der Formel (I) zu erhalten.

7. Die Verbindung der Formel (III) wie in Anspruch 6 definiert, mit dem folgenden Namen:
- [4-(1-Fluor-2-propinyl)-2,3,5,6-tetrafluor]-benzylalkohol,

8. Verwendung der Verbindungen der Formel (I) wie in irgendeinem der Ansprüche 1 bis 5 definiert, zur Herstellung von pestiziden Zusammensetzungen für die Bekämpfung von Parasiten an Pflanzen, Parasiten in Räumen und Parasiten an Warmblüter-Tieren.

9. Zusammensetzungen für die Bekämpfung von Parasiten an Pflanzen, Parasiten in Räumen und Parasiten an Warmblüter-Tieren, dadurch gekennzeichnet, daß sie als Wirkstoff mindestens eine Verbindung, wie in irgendeinem der Ansprüche 1 bis 5 definiert, umfassen.

10. Insektizide Zusammensetzungen, dadurch gekennzeichnet, daß sie als Wirkstoff mindestens eine Verbindung, wie in irgendeinem der Ansprüche 1 bis 5 definiert, umfassen.

11. Insektizide Zusammensetzungen wie in Anspruch 10 definiert, dadurch gekennzeichnet, daß sie für die Bekämpfung von DIABROTICA und anderen Bodenparasiten vorgesehen sind.

12. Akarizide Zusammensetzungen, dadurch gekennzeichnet, daß sie als Wirkstoff mindestens eine Verbindung, wie in irgendeinem der Ansprüche 1 bis 5 definiert, umfassen.

13. Zusammensetzungen wie in irgendeinem der Ansprüche 9 bis 12 definiert, dadurch gekennzeichnet, daß sie als Wirkstoff mindestens eine Verbindung, wie in Anspruch 5 definiert, umfassen.

14. Assoziationen mit insektizider, akarizider oder nematizider Wirkung, dadurch gekennzeichnet, daß sie als Wirkstoff einerseits mindestens eine der Verbindungen der allgemeinen Formel (I), wie in Anspruch 1 definiert, und andererseits mindestens einen Pyrethrinoid-Ester enthalten, gewählt aus der durch die Ester von Allethrolon, von 3,4,5,6-Tetrahydrophthalimido-methylalkohol, von 5-Benzyl-3-furyl-methylalkohol, von 3-Phenoxy-benzylalkohol und von α-Cyano-3-phenoxy-benzylalkohol mit Chrysanthemsäuren, durch die Ester von 5-Benzyl-3-furyl-methylalkohol mit 2,2-Dimethyl-3-(2-oxo-3-tetrahydrothiophenyliden-methyl)-cyclopropancarbonsäuren, durch die Ester von 3-Phenoxy-benzylalkohol und von α-Cyano-3-phenoxy-benzylalkohol mit 2,2-Dimethyl-3-(2,2-dichlorvinyl)-cyclopropancarbonsäuren, durch die Ester von α-Cyano-3-phenoxy-benzylalkohol mit 2,2-Dimethyl-3-(2,2-dibromvinyl)-cyclopropancarbonsäuren, durch die Ester von 3-Phenoxy-benzylalkohol mit 2-para-Chlorphenyl-2-isopropyl-essigsäuren, durch die Ester von Allethrolon, von 3,4,5,6-Tetrahydrophthalimido-methylalkohol, von 5-Benzyl-3-furyl-methylalkohol, von 3-Phenoxy-benzylalkohol und von α-Cyano-3-phenoxy-benzylalkohol mit 2,2-Dimethyl-3-(1,2,2,2-tetrahaloethyl)-cyclopropancarbonsäuren, worin "Halo" ein Atom von Fluor, Chlor oder Brom darstellt, gebildeten Gruppe, mit der Maßgabe, daß die Verbindungen (I) in allen möglichen stereoisomeren Formen existieren können, ebenso wie die Paare von Säuren und Alkoholen der oben genannten Pyrethrinoid-Ester.

## Patentansprüche (Patentansprüche für folgende(n) Vertragsstaat(en): ES)

1. Verfahren zur Herstellung von allen möglichen stereoisomeren Formen sowie ihren Mischungen der Verbindungen der allgemeinen Formel (I) 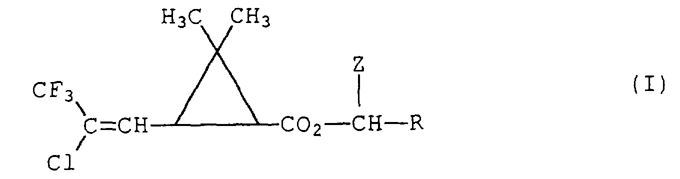 in der
Z ein Wasserstoffatom, einen Rest CH₃, einen Rest C≡N oder einen Rest C≡CH darstellt,
und R einen substituierten Phenylrest bedeutet, gewählt aus der durch die folgenden Reste gebildeten Gruppe: 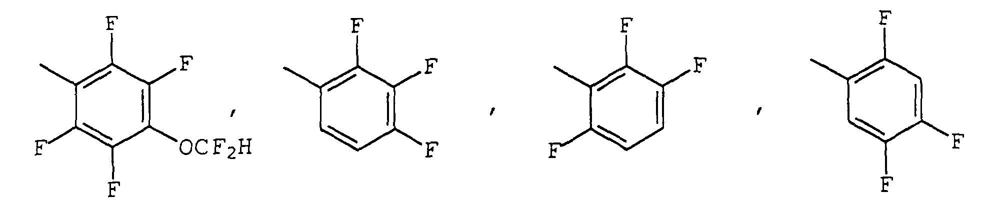 und 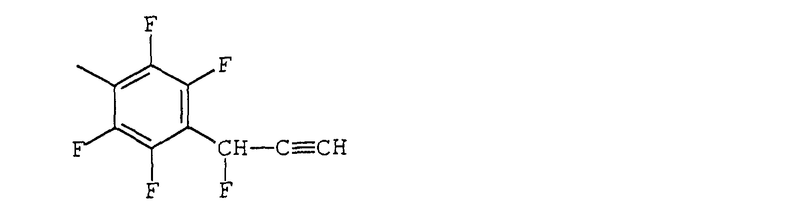 dadurch gekennzeichnet, daß man eine Säure der Formel (II) 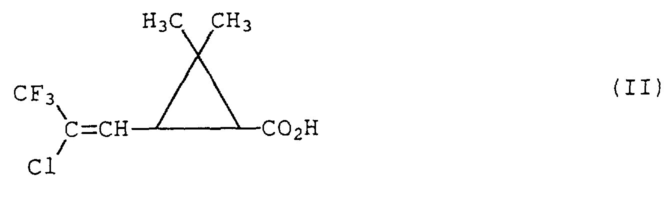 oder ein funktionelles Derivat dieser Säure der Einwirkung eines Alkohols der Formel (III) 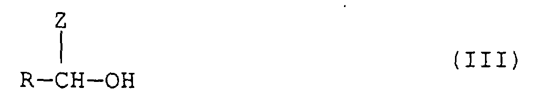 in der R und Z ihre oben angegebene Bedeutung behalten, oder eines funktionellen Derivates dieses Alkohols unterzieht, um die entsprechende Verbindung der Formel (I) zu erhalten.

2. Verfahren nach Anspruch 1, dadurch gekennzeichnet, daß man als Ausgangsprodukt eine Säure der Formel (II) verwendet, in der die Cyclopropan-Struktur die Struktur 1R [cis] besitzt.

3. Verfahren nach Anspruch 1 oder 2, dadurch gekennzeichnet, daß man als Ausgangsprodukt eine Säure der Formel (II) verwendet, in der die Geometrie der Doppelbindung Z ist.

4. Verfahren nach irgendeinem der Ansprüche 1 bis 3, dadurch gekennzeichnet, daß man als Ausgangsprodukt einen Alkohol der Formel (III) verwendet, in der Z ein Wasserstoffatom darstellt.

5. Verfahren nach irgendeinem der Ansprüche 1 bis 4, dadurch gekennzeichnet, daß man die 1R [1α,3α(Z)]-2,2-Dimethyl-3-cyclopropancarbonsäure der Einwirkung eines geeigneten Alkohols der Formel (III) oder eines funktionellen Derivates dieses Alkohols unterzieht, um eine der Verbindungen der Formel (I) zu erhalten, von denen die Namen folgen:
- 1R[1α,3α(Z)]-2,2-Dimethyl-3-(3,3,3-trifluor-2-chlor-propenyl)-cyclopropancarbonsäure-[4-(difluormethoxy)-2,3,5,6-tetrafluorphenyl]-methylester,
- 1R[1α,3α(Z)]-2,2-Dimethyl-3-(3,3,3-trifluor-2-chlor-propenyl)-cyclopropancarbonsäure-(2,3,4-trifluorphenyl)-methylester,
- 1R[1α,3α(Z)]-2,2-Dimethyl-3-(3,3,3-trifluor-2-chlor-propenyl)-cyclopropancarbonsäure-(2,3,6-trifluorphenyl)-methylester,
- 1R[1α,3α(Z)]-2,2-Dimethyl-3-(3,3,3-trifluor-2-chlor-propenyl)-cyclopropancarbonsäure-(2,4,5-trifluorphenyl)-methylester,
- 1R[1α,3α(Z)]-2,2-Dimethyl-3-(3,3,3-trifluor-2-chlor-propenyl)-cyclopropancarbonsäure-{[4-(1-fluor-2-propinyl)]-2,3,5,6-tetrafluorphenyl}-methylester.

## Patentansprüche (Patentansprüche für folgende(n) Vertragsstaat(en): PT)

1. Verfahren zur Herstellung von allen möglichen stereoisomeren Formen sowie ihren Mischungen der Verbindungen der allgemeinen Formel (I) 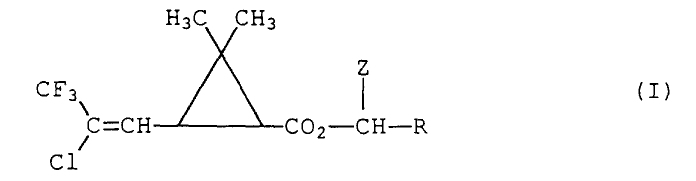 in der
Z ein Wasserstoffatom, einen Rest CH₃, einen Rest C≡N oder einen Rest C≡CH darstellt,
und R einen substituierten Phenylrest bedeutet, gewählt aus der durch die folgenden Reste gebildeten Gruppe: 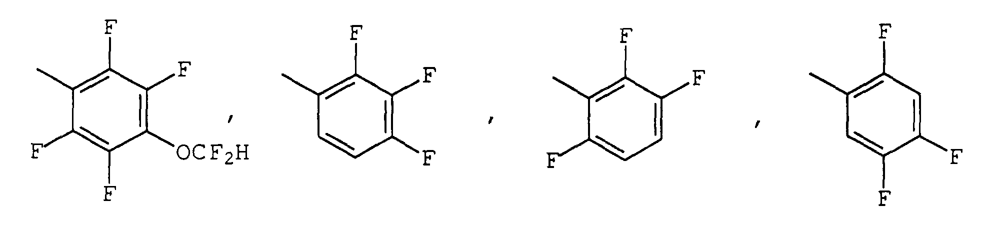 und 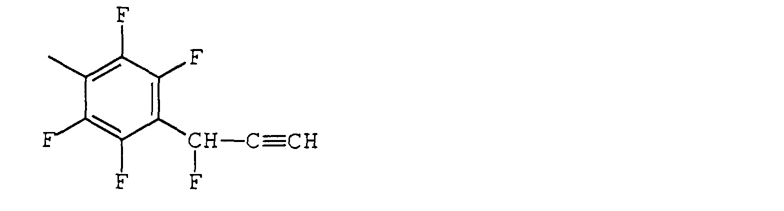 dadurch gekennzeichnet, daß man eine Säure der Formel (II) 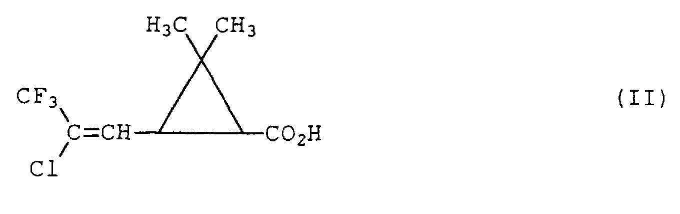 oder ein funktionelles Derivat dieser Säure der Einwirkung eines Alkohols der Formel (III) 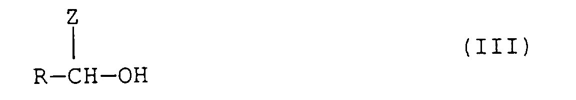 in der R und Z ihre oben angegebene Bedeutung behalten, oder eines funktionellen Derivates dieses Alkohols unterzieht, um die entsprechende Verbindung der Formel (I) zu erhalten.

2. Verfahren nach Anspruch 1, dadurch gekennzeichnet, daß man als Ausgangsprodukt eine Säure der Formel (II) verwendet, in der die Cyclopropan-Struktur die Struktur 1R [cis] besitzt.

3. Verfahren nach Anspruch 1 oder 2, dadurch gekennzeichnet, daß man als Ausgangsprodukt eine Säure der Formel (II) verwendet, in der die Geometrie der Doppelbindung Z ist.

4. Verfahren nach irgendeinem der Ansprüche 1 bis 3, dadurch gekennzeichnet, daß man als Ausgangsprodukt einen Alkohol der Formel (III) verwendet, in der Z ein Wasserstoffatom darstellt.

5. Verfahren nach irgendeinem der Ansprüche 1 bis 4, dadurch gekennzeichnet, daß man die 1R[1α,3α(Z)]-2,2-Dimethyl-3-cyclopropancarbonsäure der Einwirkung eines geeigneten Alkohols der Formel (III) oder eines funktionellen Derivates dieses Alkohols unterzieht, um eine der Verbindungen der Formel (I) zu erhalten, von denen die Namen folgen:
- 1R[1α,3α(Z)]-2,2-Dimethyl-3-(3,3,3-trifluor-2-chlor-propenyl)-cyclopropancarbonsäure-[4-(difluormethoxy)-2,3,5,6-tetrafluorphenyl]-methylester,
- 1R[1α,3α(Z)]-2,2-Dimethyl-3-(3,3,3-trifluor-2-chlor-propenyl)-cyclopropancarbonsäure-(2,3,4-trifluorphenyl)-methylester,
- 1R[1α,3α(Z)]-2,2-Dimethyl-3-(3,3,3-trifluor-2-chlor-propenyl)-cyclopropancarbonsäure-(2,3,6-trifluorphenyl)-methylester,
- 1R[1α,3α(Z)]-2,2-Dimethyl-3-(3,3,3-trifluor-2-chlor-propenyl)-cyclopropancarbonsäure-(2,4,5-trifluorphenyl)-methylester,
- 1R[1α,3α(Z)]-2,2-Dimethyl-3-(3,3,3-trifluor-2-chlor-propenyl)-cyclopropancarbonsäure-{[4-(1-fluor-2-propinyl)]-2,3,5,6-tetrafluorphenyl}-methylester.

6. Verwendung der Verbindungen der Formel (I) wie in irgendeinem der Ansprüche 1 bis 5 definiert, zur Bekämpfung von Parasiten an Pflanzen, Parasiten in Räumen und Parasiten an Warmblüter-Tieren.

7. Verfahren zur Herstellung von Zusammensetzungen für die Bekämpfung von Parasiten an Pflanzen, Parasiten in Räumen und Parasiten an Warmblüter-Tieren, dadurch gekennzeichnet, daß man mindestens eine der Verbindungen der Formel (I), wie in irgendeinem der Ansprüche 1 bis 5 definiert, in eine für diese Anwendung vorgesehene Form bringt.

8. Verfahren zur Herstellung von insektiziden Zusammensetzungen, dadurch gekennzeichnet, daß man mindestens eine der Verbindungen der Formel (I), wie in irgendeinem der Ansprüche 1 bis 5 definiert, in eine für diese Anwendung vorgesehene Form bringt.

9. Verfahren zur Herstellung von insektiziden Zusammensetzungen, vorgesehen für die Bekämpfung von DIABROTICA und anderen Bodenparasiten, dadurch gekennzeichnet, daß man mindestens eine der Verbindungen der Formel (I), wie in irgendeinem der Ansprüche 1 bis 5 definiert, in eine für diese Anwendung vorgesehene Form bringt.

10. Verfahren zur Herstellung von akariziden Zusammensetzungen, dadurch gekennzeichnet, daß man mindestens eine der Verbindungen der Formel (I), wie in irgendeinem der Ansprüche 1 bis 5 definiert, in eine für diese Anwendung vorgesehene Form bringt.

11. Verfahren zur Herstellung von Zusammensetzungen nach irgendeinem der Ansprüche 7 bis 10, dadurch gekennzeichnet, daß man mindestens eine der Verbindungen der Formel (I), wie in Anspruch 5 definiert, in eine für diese Anwendung vorgesehene Form bringt.

12. Verfahren zur Herstellung von Zusammensetzungen mit insektizider, akarizider oder nematizider Wirkung, dadurch gekennzeichnet, daß man mindestens eine der Verbindungen der allgemeinen Formel (I), wie in irgendeinem der Ansprüche 1 bis 5 definiert, mit mindestens einem Pyrethrinoid-Ester mischt, gewählt aus der durch die Ester von Allethrolon, von 3,4,5,6-Tetrahydrophthal-imido-methylalkohol, von 5-Benzyl-3-furyl-methylalkohol, von 3-Phenoxy-benzylalkohol und von α-Cyano-3-phenoxy-benzylalkohol mit Chrysanthemsäuren, durch die Ester von 5-Benzyl-3-furyl-methylalkohol mit 2,2-Dimethyl-3-(2-oxo-3-tetrahydrothiophenylidenmethyl)-cyclopropancarbonsäuren, durch die Ester von 3-Phenoxy-benzylalkohol und von α-Cyano-3-phenoxy-benzylalkohol mit 2,2-Dimethyl-3-(2,2-dichlorvinyl)-cyclopropancarbonsäuren, durch die Ester von α-Cyano-3-phenoxy-benzylalkohol mit 2,2-Dimethyl-3-(2,2-dibromvinyl)-cyclopropancarbonsäuren, durch die Ester von 3-Phenoxy-benzylalkohol mit 2-para-Chlorphenyl-2-isopropyl-essigsäuren, durch die Ester von Allethrolon, von 3,4,5,6-Tetrahydrophthalimido-methylalkohol, von 5-Benzyl-3-furyl-methylalkohol, von 3-Phenoxy-benzylalkohol und von α-Cyano-3-phenoxy-benzylalkohol mit 2,2-Dimethyl-3-(1,2,2,2-tetrahaloethyl)-cyclopropancarbonsäuren, worin "Halo" ein Atom von Fluor, Chlor oder Brom darstellt, gebildeten Gruppe, mit der Maßgabe, daß die Verbindungen (I) in allen möglichen stereoisomeren Formen existieren können, ebenso wie die Paare von Säuren und Alkoholen der oben genannten Pyrethrinoid-Ester.

## Claims (Claims for the following Contracting State(s): AT, BE, CH, DE, DK, FR, GB, GR, IT, LI, LU, MC, NL, SE)

1. In all the possible stereoisomer forms as well as their mixtures, the compounds corresponding to the general formula (I): 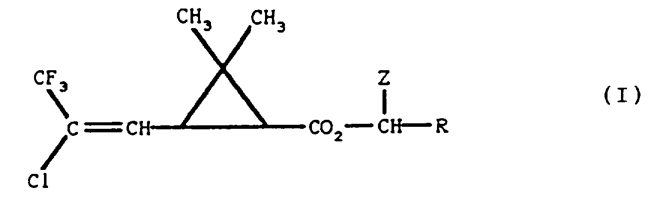 in which:
Z represents a hydrogen atom, a CH₃ radical, a C≡N radical, or a C≡CH radical,
and R represents a substituted phenyl radical chosen from the group constituted by the following radicals: 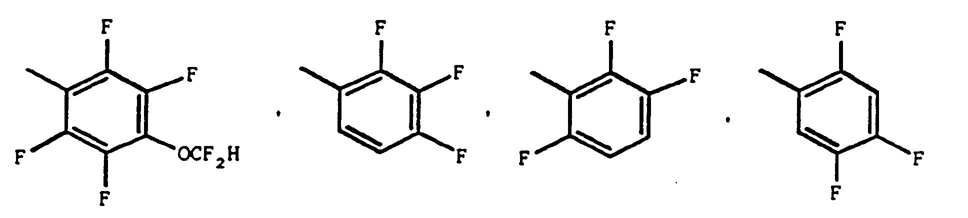 ET

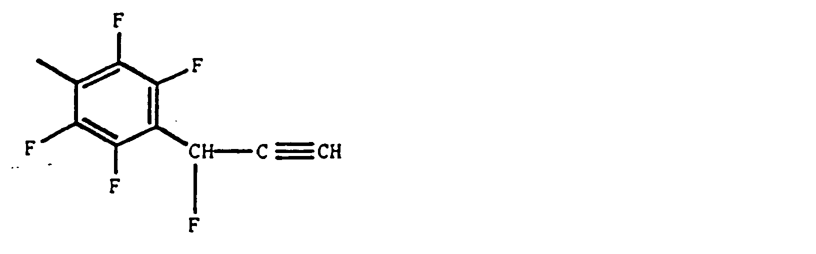

2. The compounds of formula (I) as defined in claim 1 in which the cyclopropane structure is of 1R [cis] structure.

3. The compounds of formula (I) according to claim 1 in which the geometry of the double bond is Z.

4. The compounds of formula (I) according to claim 1 in which Z represents a hydrogen atom.

5. The compounds of formula (I) as defined in claim 1 of which the names follow:
- [4-(difluoromethoxy) 2,3,5,6-tetrafluorophenyl] methyl 1R [1alpha, 3alpha (Z)] 2,2-dimethyl 3-(3,3,3-trifluoro 2-chloro propenyl) cyclopropanecarboxylate,
- (2,3,4-trifluorophenyl) methyl 1R [1alpha, 3alpha (Z)] 2,2-dimethyl 3-(3,3,3-trifluoro 2-chloro propenyl) cyclopropanecarboxylate,
- (2,3,6-trifluorophenyl) methyl 1R [1alpha, 3alpha (Z)] 2,2-dimethyl 3-(3,3,3-trifluoro 2-chloro propenyl) cyclopropanecarboxylate,
- (2,4,5-trifluorophenyl) methyl 1R [1alpha, 3alpha (Z)] 2,2- dimethyl 3-(3,3,3-trifluoro 2-chloro propenyl) cyclopropanecarboxylate,
- [[(4-(1-fluoro 2-propynyl)] 2,3,5,6-tetrafluoro phenyl] methyl 1R [1alpha, 3alpha (Z)] 2,2-dimethyl 3-(3,3,3-trifluoro 2-chloro propenyl) cyclopropanecarboxylate,
- (2,3,6-trifluorophenyl) methyl 1R [1alpha, 3alpha (E + Z)] 2,2-dimethyl-3-(3,3,3-trifluoro-2-chloropropenyl) cyclopropanecarboxylate.

6. Preparation process for the compounds of formula (I) as defined in any one of claims 1 to 5, characterized in that an acid of formula (II): 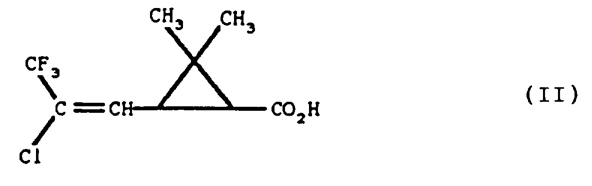 or a functional derivative of this acid is subjected to the action of an alcohol of formula (III): 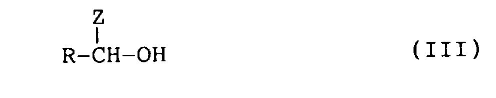 in which R and Z retain their previous meaning, or a functional derivative of this alcohol in order to obtain the corresponding compound of formula (I).

7. The compound of formula (III) as defined in claim 6 of which the name follows:
- [4-(1-fluoro 2-propynyl) 2,3,5,6-tetrafluoro] benzyl alcohol.

8. Use of the compounds of formula (I) as defined in any one of claims 1 to 5 for the preparation of pesticide compositions intended for combating parasites of vegetation, parasites of premises and parasites of warm-blooded animals.

9. The compositions intended for combating parasites of vegetation, parasites of premises and parasites of warm-blooded animals, characterized in that they contain as active ingredient at least one compound defined in any one of claims 1 to 5.

10. The insecticide compositions, characterized in that they contain as active ingredient at least one compound defined in any one of claims 1 to 5.

11. The insecticide compositions defined in claim 10, characterized in that they are intended for combating DIABROTICA and other soil parasites.

12. The acaricide compositions, characterized in that they contain as active ingredient at least one compound defined in any one of claims 1 to 5.

13. The compositions defined in any one of claims 9 to 12 characterized in that they contain as active ingredient at least one compound defined in claim 5.

14. Combinations endowed with insecticide, acaricide or nematicide activity, characterized in that they contain as active ingredient, on the one hand, at least one of the compounds of general formula (I) as defined in claim 1 and on the other hand, at least one of the pyrethrinoid esters chosen from the group constituted by the esters of allethrolone, of 3,4,5,6-tetrahydrophthalimido methyl alcohol, of 5-benzyl-3-furyl methyl alcohol, of 3-phenoxybenzyl alcohol and of alpha-cyano-3-phenoxy benzyl alcohols with chrysanthemic acids, by the esters of 5-benzyl-3-furyl methyl alcohol with 2,2-dimethyl-3-(2-oxo-3-tetrahydrothiophenylidene methyl)-cyclopropanecarboxylic acids, by the esters of 3-phenoxy benzyl alcohol and of alpha-cyano-3-phenoxy benzyl alcohol with 2,2-dimethyl-3-(2,2-dichlorovinyl)-cyclopropanecarboxylic acids, by the esters of alpha-cyano-3-phenoxy benzyl alcohol with 2,2-dimethyl-3-(2,2-dibromovinyl)-cyclopropanecarboxylic acids, by the esters of 3-phenoxy benzyl alcohol with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxy benzyl alcohol, and of alpha-cyano-3-phenoxy benzyl alcohol with 2,2-dimethyl-3-(1,2,2,2-tetrahaloethyl)-cyclopropanecarboxylic acids, in which "halo" represents a fluorine, chlorine or bromine atom, it being understood that the compounds (I) can exist in all their possible stereoisomer forms as well as the acid and alcohol copulas of the above pyrethrinoid esters.

## Claims (Claims for the following Contracting State(s): PT)

1. Process for preparing, in all the possible stereoisomer forms as well as their mixtures, the compounds corresponding to the general formula (I): 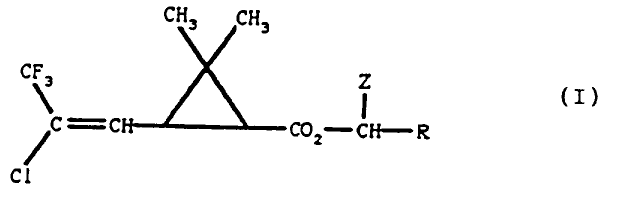 in which:
Z represents a hydrogen atom, a CH₃ radical, a C≡N radical, or a C≡CH radical,
and R represents a substituted phenyl radical chosen from the group constituted by the following radicals: 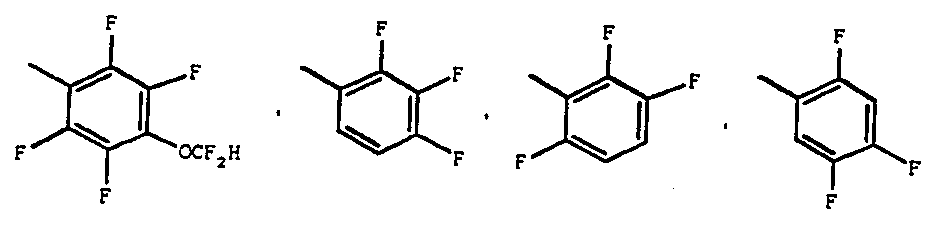 ET 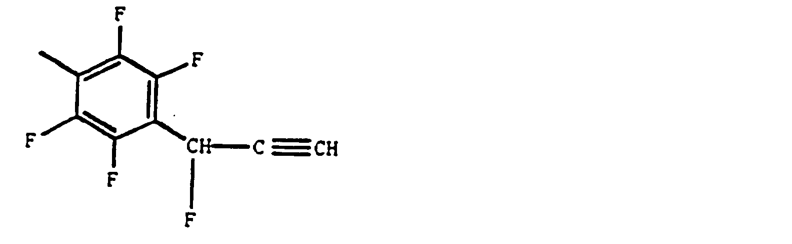 characterized in that an acid of formula (II): 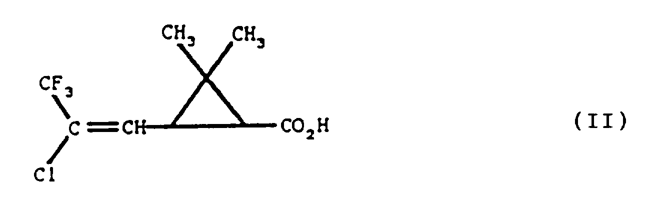 or a functional derivative of this acid is subjected to the action of an alcohol of formula (III): 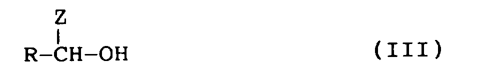 in which R and Z retain their previous meaning, or a functional derivative of this alcohol in order to obtain the corresponding compound of formula (I).

2. Process according to claim 1, characterized in that an acid of formula (II) is used at the start in which the cyclopropane structure is of 1R [cis] structure.

3. Process according to claim 1 or 2, characterized in that an acid of formula (II) is used at the start in which the geometry of the double bond is Z.

4. Process according to any one of claims 1 to 3, characterized in that an alcohol of formula (III) is used at the start in which Z represents a hydrogen atom.

5. Process according to any one of claims 1 to 4, characterized in that 1R [1alpha, 3alpha (Z)] 2,2-dimethyl 3-cyclopropane carboxylic acid is subjected to the action of a suitable alcohol of formula (III) or a functional derivative of this alcohol in order to obtain one of the compounds of formula (I) of which the names follow:
- [4-(difluoromethoxy) 2,3,5,6-tetrafluorophenyl] methyl 1R [1alpha, 3alpha (Z)] 2,2-dimethyl 3-(3,3,3-trifluoro 2-chloro propenyl) cyclopropanecarboxylate,
- (2,3,4-trifluorophenyl) methyl 1R [1alpha, 3alpha (Z)] 2,2-dimethyl 3-(3,3,3-trifluoro 2-chloro propenyl) cyclopropanecarboxylate,
- (2,3,6-trifluorophenyl) methyl 1R [1alpha, 3alpha (Z)] 2,2-dimethyl 3-(3,3,3-trifluoro 2-chloro propenyl) cyclopropanecarboxylate,
- (2,4,5-trifluorophenyl) methyl 1R [1alpha, 3alpha (Z)] 2,2-dimethyl 3-(3,3,3-trifluoro 2-chloro propenyl) cyclopropanecarboxylate,
- [[(4-(1-fluoro 2-propynyl)] 2,3,5,6-tetrafluoro phenyl] methyl 1R [1alpha, 3alpha (Z)] 2,2-dimethyl 3-(3,3,3-trifluoro 2-chloro propenyl) cyclopropanecarboxylate.

## Claims (Claims for the following Contracting State(s): ES)

1. Process for preparing, in all the possible stereoisomer forms as well as their mixtures, the compounds corresponding to the general formula (I): 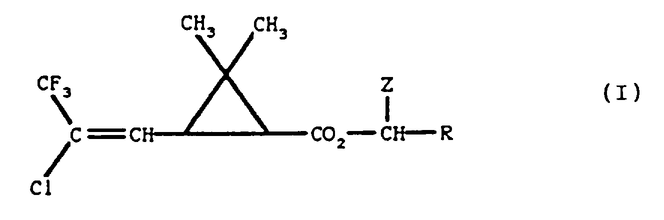 in which:
Z represents a hydrogen atom, a CH₃ radical, a C≡N radical, or a C≡CH radical,
and R represents a substituted phenyl radical chosen from the group constituted by the following radicals: 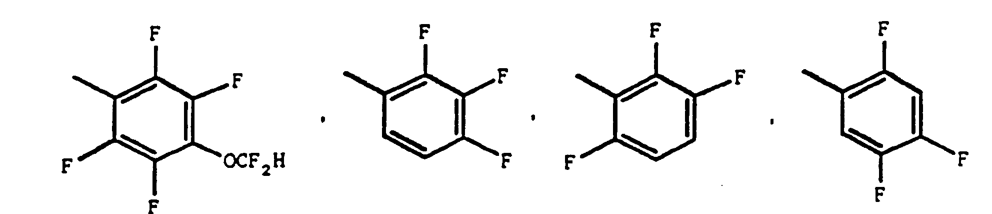 ET 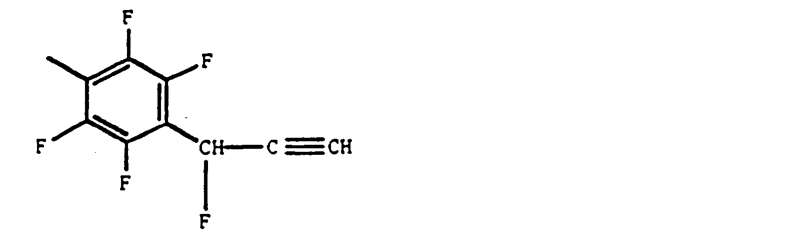 characterized in that an acid of formula (II): 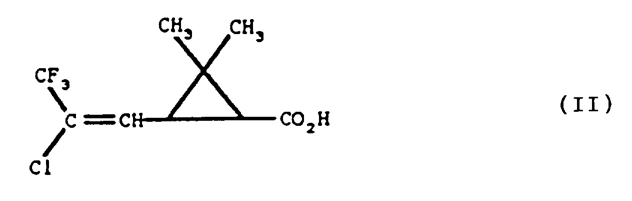 or a functional derivative of this acid is subjected to the action of an alcohol of formula (III): 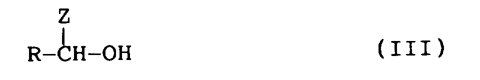 in which R and Z retain their previous meaning, or a functional derivative of this alcohol in order to obtain the corresponding compound of formula (I).

2. Process according to claim 1, characterized in that an acid of formula (II) is used at the start in which the cyclopropane structure is of 1R [cis] structure.

3. Process according to claim 1 or 2, characterized in that an acid of formula (II) is used at the start in which the geometry of the double bond is Z.

4. Process according to any one of claims 1 to 3, characterized in that an alcohol of formula (III) is used at the start in which Z represents a hydrogen atom.

5. Process according to any one of claims 1 to 4, characterized in that 1R [1alpha, 3alpha (Z)] 2,2-dimethyl 3-cyclopropane carboxylic acid is subjected to the action of a suitable alcohol of formula (III) or a functional derivative of this alcohol in order to obtain one of the compounds of formula (I) of which the names follow:
- [4-(difluoromethoxy) 2,3,5,6-tetrafluorophenyl] methyl 1R [1alpha, 3alpha (Z)] 2,2-dimethyl 3-(3,3,3-trifluoro 2-chloro propenyl) cyclopropanecarboxylate,
- (2,3,4-trifluorophenyl) methyl 1R [1alpha, 3alpha (Z)] 2,2-dimethyl 3-(3,3,3-trifluoro 2-chloro propenyl) cyclopropanecarboxylate,
- (2,3,6-trifluorophenyl) methyl 1R [1alpha, 3alpha (Z)] 2,2-dimethyl 3-(3,3,3-trifluoro 2-chloro propenyl) cyclopropanecarboxylate,
- (2,4,5-trifluorophenyl) methyl 1R [1alpha, 3alpha (Z)] 2,2-dimethyl 3-(3,3,3-trifluoro 2-chloro propenyl) cyclopropanecarboxylate,
- [[(4-(1-fluoro 2-propynyl)] 2,3,5,6-tetrafluoro phenyl] methyl 1R [1alpha, 3alpha (Z)] 2,2-dimethyl 3-(3,3,3-trifluoro 2-chloro propenyl) cyclopropanecarboxylate.

6. Use of the compounds of formula (I) as defined in any one of claims 1 to 5, for combating parasites of vegetation, parasites of premises and parasites of warm-blooded animals.

7. Process for preparing compositions intended for combating parasites of vegetation, of premises and of warm-blooded animals, characterized in that at least one of the compounds of formula (I) as defined in any one of claims 1 to 5 is put in a form intended for this use.

8. Process for preparing insecticide compositions, characterized in that at least one of the compounds of formula (I) as defined in any one of claims 1 to 5 is put in a form intended for this use.

9. Process for preparing insecticide compositions, intended for combating Diabrotica and other soil parasites, characterized in that at least one of the compounds of formula (I) as defined in any one of claims 1 to 5 is put in a form intended for this use.

10. Process for preparing acaricide compositions, characterized in that at least one of the compounds of formula (I) as defined in any one of claims 1 to 5 is put in a form intended for this use.

11. Process for preparing compositions according to any one of claims 7 to 10, characterized in that at least one of the compounds of formula (I) as defined in claim 5 is put in a form intended for this use.

12. Preparation process for compositions endowed with insecticide, acaricide or nematicide activity, characterized in that at least one of the compounds of formula (I) as defined in any one of claims 1 to 5 is mixed with at least one of the pyrethrinoid esters chosen from the group constituted by the esters of allethrolone, of 3,4,5,6-tetrahydrophthalimido methyl alcohol, of 5-benzyl-3-furyl methyl alcohol, of 3-phenoxy benzyl alcohol and of alpha-cyano-3-phenoxy benzyl alcohols with chrysanthemic acids, by the esters of 5-benzyl-3-furyl methyl alcohol with 2,2-dimethyl-3-(2-oxo-3-tetrahydrothiophenylidene methyl)-cyclopropanecarboxylic acids, by the esters of 3-phenoxy benzyl alcohol and of alpha-cyano-3-phenoxy benzyl alcohol with 2,2-dimethyl-3-(2,2-dichlorovinyl)-cyclopropanecarboxylic acids, by the esters of alpha-cyano-3-phenoxy benzyl alcohol with 2,2-dimethyl-3-(2,2-dibromovinyl)-cyclopropanecarboxylic acids, by the esters of 3-phenoxy benzyl alcohol with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxy benzyl alcohol, and of alpha-cyano-3-phenoxy benzyl alcohol with 2,2-dimethyl-3-(1,2,2,2-tetrahaloethyl)-cyclopropanecarboxylic acids, in which "halo" represents a fluorine, chlorine or bromine atom, it being understood that the compounds (I) can exist in all their possible stereoisomer forms as well as the acid and alcohol copulas of the above pyrethrinoid esters.
